# EUROPEAN PATENT SPECIFICATION

(11) **EP 2 088 987 B1**
(45) Date of publication and mention of the grant of the patent: **30.05.2012**
(21) Application number: 07839917.7
(22) Date of filing: 02.11.2007
(51) Int. Cl.: A61K 8/19, A61K 8/28, A61K 8/29, A61K 8/73, A61Q 5/12

(54) **DISPERSIBLE NON-BORATE METAL SALT OR CHELATE TREATED POLYGALACTOMANNAN POLYMERS FOR USE IN PERSONAL CARE AND HOUSEHOLD CARE APPLICATIONS**
DISPERGIERBARE NICHT-BORAT-METALLSALZ- ODER CHELAT-BEHANDELTE POLYGALACTOMANNAN-POLYMERE ZUR VERWENDUNG IN KÖRPERPFLEGE- UND HAUSHALTSPFLEGEANWENDUNGEN
POLYMÈRES DISPERSIBLES DE POLYGALACTOMANNANE TRAITÉS PAR UN SEL OU CHÉLATE MÉTALLIQUE NON-BORATE POUR UNE UTILISATION DANS DES APPLICATIONS DE SOINS CORPORELS ET D'ENTRETIEN MÉNAGER

(30) Priority: 03.11.2006 US 856486 P
(43) Date of publication of application: 19.08.2009
(73) Proprietor: Hercules Incorporated, Wilmington, DE 19894-0001 (US)
(72) Inventor: ERAZO-MAJEWICZ, Paquita, Landenberg, PA 19350 (US); KROON, Gijsbert, 3371 BW Hardinxveld Giessendam (NL); MAJEWICZ, Thomas G., Landenberg, PA 19350 (US)
(74) Representative: HOFFMANN EITLE
(86) International application number: PCT/US2007/023157
(87) International publication number: WO 2008/076178

(56) References cited:
- EP-A- 0 586 235
- WO-A-2006/106366
- FR-A- 2 513 265
- US-A- 4 645 833
- US-A- 4 654 158
- US-A- 4 677 201
- US-A- 5 670 141
- US-A1- 2005 227 902

## Description

### FIELD OF THE INVENTION

The present invention relates to the use of a cationic guar gum which is crosslinked with a non-borate titanate or zirconate organic chelating agent to form discrete guar particles which are capable of being easily dispersed in water which permits subsequent processing of the guar such as washing. The guar and its derivative are used for personal care or household care.

### BACKGROUND OF THE INVENTION

Polygalactomannans and their derivatives are used in various applications such as oil recovery, personal care products, textile applications, paper applications, coating applications, food applications, etc. Polygalactomannans and their derivatives are difficult to disperse in aqueous solutions, as they tend to form sticky particles which clump and agglomerate, making dissolution difficult. To improve dissolution of the polymers, crosslinking agents, such as borax, are used to allow for water-washing of the polygalactomannan after reaction and for improved dispersibility of the polygalactomannan in water.

Crosslinking agents based on borate salts, aluminum salts, copper, iron, lead, calcium, and sodium salts have been described. Other crosslinking agents such as metal salts based on titanium and zirconium have been mentioned, without clear definition of the method or procedure for their use.

There exists a concern over the hazards of boron in some consumer products, and a need exists for alternative crosslinking agents for use in the purification and handling of polygalactomannan polymers and their derivatives.

In personal care applications, such as in hair care and skincare, and in household applications, such as fabric care applications, there is a desire to deposit a boron free coating onto the substrate, that reduces the energy needed to move a comb through hair in the wet or dry state or delivers a silky, soft feel to skin or to fabric. This coating can also act to improve the luster and moisture retention of hair and skin, as well as their manageability and feel.

### SUMMARY OF THE INVENTION

The present invention is directed to the use of a non-borate titanium or zirconium chelate crosslinked guar containing cationic substituents for personal care or household care. This low impurity content guar is of utility in various applications such as personal care and household care products. The low impurity content guar is of particular utility in applications where the use of boron containing compounds is to be reduced or eliminated.

### DETAILED DESCRIPTION OF THE INVENTION

In the present invention, it has been found that treatment of cationic guar reaction mixture with titanium salts at a ratio of between about 0.001-0.025 wt titanium/wt guar polymer leads to a water-dispersible guar that remains as a discrete particulate that does not agglomerate into a gel. Other chelates, such as zirconium salts are expected to give a similar result as the titanium salts. The resulting crosslinked guar particle is easily washed in water and readily dispersible in water.

This application is of utility as a processing aid for galactomannans. Polygalactomannans are polysaccharides composed principally of galactose and mannose units and are usually found in the endosperm of leguminous seeds such as guar, locust bean, honey locust, flame tree, and the like. The polygalactomannans may be used in either their natural form or may be substituted with one or more functional groups (e.g., carboxymethyl group). The most commonly used polygalactomannan is guar. The guar used in the present invention is derivatized guar, namely cationic guar.

An advantage of the use of titanate chelates over borate salts is that borate salts disassociate in water at a higher pH than titanate chelates. This results in the formation of swelled gel particles or a gel mass at a higher pH. By permitting the polygalactomannan to remain as a discrete particle at lower pH values, washing of the polygalactomannan over a greater pH range may be performed and thereby permitting removal of impurities that would not be removed at the higher pH ranges, such as alkaline species. These impurities may reduce the clarity of aqueous solutions of the polygalactomannan or be harmful if permitted to remain with the galactomannan in certain end use applications, such as personal care.

In addition, it has been found that titanium chelate treated cationic guars function well as conditioning agents and thickening agents in personal care compositions.

In accordance with the present invention, the cationic guar has a substituent degree of substitution (DS) lower limit of 0.001 and an upper limit of 3.0. Preferably, the lower limit of the cationic DS is 0.01, and more preferably 0.05. The upper limit of the cationic DS is 3.0, preferably 1.0, and more preferably 0.25. The cationic guar of the present invention generally has a weight average molecular weight (Mw) with a lower limit of about 50,000 and an upper limit of about 5,000,000 preferably, the lower limit of the molecular weight is 300,000, and more preferably 400,000. Preferably, the upper limit of the molecular weight is 1,500,000, more preferably 1,000,000.

The cationic functionality of the guar or derivatized guar can be added to the backbone by known methods. For example, the guar material can be reacted for a sufficient time and at a sufficient temperature with tertiary amino or quaternary ammonium alkylating reagents, such 2-dialkylaminoethyl chloride and quaternary ammonium compounds such as 3-chloro-2-hydroxypropyltrimethylammonium chloride, and 2,3-epoxy-propyltrimethylammonium chloride. Preferred examples include glycidyltrialkylammonium salts and 3-halo-2-hydroxypropyltrialkylammonium salts such as glycidyltrimethylammonium chloride, glycidyltriethylammonium chloride, gylcidyltripropylammonium chloride, glycidylethyldimethylammonium chloride, glycidyldiethylmethylammonium chloride, and their corresponding bromides and iodides; 3-chloro-2-hydroxypropyltrimethylammonium chloride, 3-chloro-2-hydroxypropyltriethylammonium chloride, 3-chloro-2-hydroxypropyltripropylammonium chloride, 3-chloro-2-hydroxypropylethyldimethylammonium chloride, and their corresponding bromides and iodides; and quaternary ammonium compounds such as halides of imidazoline ring containing compounds.

The cationic guar may also contain other substituent groups such as nonionic substituents, i.e., hydroxyalkyl wherein the alkyl represents a straight or branched hydrocarbon moiety having 1 to 30 carbon atoms (e.g., hydroxyethyl, hydroxypropyl, hydroxybutyl) or anionic substituents, such as carboxymethyl groups are optional. These optional substituents are linked to the guar polymer by the reaction with reagents such as (1) alkylene oxides (e.g., ethylene oxide, propylene oxide, butylene oxide) to obtain hydroxyethyl groups, hydroxypropyl groups, or hydroxybutyl groups, or with (2) chloromethyl acetic acid to obtain a carboxymethyl group. The process for preparing derivatized guar is well known in the art. The cationic guar may also contain mixture of one or more other substituent groups such as nonionic, anionic and cationic substituents.

Cationic guar polymers or their derivatives, useful in the invention can be treated with several known reagents, such as (1) caustic, (2) acids, (3) by biochemical oxidants, such as galactose oxidase, (4) chemical oxidants, such as hydrogen peroxide, (5) a physical method using high speed agitation and shearing machines, (6) thermal methods, (7) enzymatic reagents, and (8) mixtures of these reagents and methods. Reagents such as sodium metabisulfite or inorganic salts of bisulfite may also be optionally included.

The end-use of the organic metal chelate treated cationic polygalactomannan polymers is as a component in personal care compositions and household care compositions, where the personal care composition comprises a organic titanium chelate treated cationic guar and a personal care ingredient. The personal care ingredient includes, but is not limited to, active ingredients, such as for example analgesics, anesthetics, antibiotic agents, antifungal agents, antiseptic agents, antidandruff agents, antibacterial agents, vitamins, hormones, antidiarrhea agents, corticosteroids, anti-inflammatory agents, vasodilators, kerolytic agents, dry-eye compositions, wound-healing agents, anti-infection agents, as well as solvents, diluents, adjuvants and other ingredients such as water, ethyl alcohol, isopropyl alcohol, propylene glycol, higher alcohols, glycerine, sorbitol, mineral oil, preservatives, surfactants, propellants, fragrances, essential oils, and viscosifying agents.

In accordance with the present invention, the personal care ingredient must provide some benefit to the user's body. Personal care compositions include hair care, skincare, sun care, and oral care compositions. Examples of substances that may suitably be included, but not limited to, in the personal care products according to the present invention are as follows:
1) Perfumes, which give rise to an olfactory response in the form of a fragrance and deodorant perfumes which in addition to providing a fragrance response can also reduce body malodor;
2) Skin coolants, such as menthol, menthyl acetate, menthyl pyrrolidone carboxylate N-ethyl-p-menthane-3-carboxamide and other derivatives of menthol, which give rise to a tactile response in the form of a cooling sensation on the skin;
3) Emollients, such as isopropylmyristate, silicone materials, mineral oils and vegetable oils which give rise to a tactile response in the form of an increase in skin lubricity;
4) Deodorants other than perfumes, whose function is to reduce the level of or eliminate micro flora at the skin surface, especially those responsible for the development of body malodor. Precursors of deodorants other than perfume can also be used;
5) Antiperspirant actives, whose function is to reduce or eliminate the appearance of perspiration at the skin surface;
6) Moisturizing agents, that keep the skin moist by either adding moisture or preventing from evaporating from the skin;
7) Cleansing agents, that remove dirt and oil from the skin;
8) Sunscreen active ingredients, which protect the skin and hair from UV and other harmful light rays from the sun. In accordance with this invention a therapeutically effective amount will normally be from 0.01 to 10% by weight, preferable 0.1 to 5% by weight of the composition;
9) Hair treatment agents, that condition the hair, cleanse the hair, detangles hair, acts as styling agent, volumizing and gloss agents, color retention agent, anti-dandruff agent, hair growth promoters, hair dyes and pigments, hair perfumes, hair relaxer, hair bleaching agent, hair moisturizer, hair oil treatment agent, and antifrizzing agent;
10) Oral care agents, such as dentifrices and mouth washes, that clean, whiten, deodorize and protect the teeth and gum;
11) Denture adhesives that provide adhesion properties to dentures;
12) Shaving products, such as creams, gels and lotions and razor blade lubricating strips;
13) Tissue paper products, such as moisturizing or cleansing tissues;
14) Beauty aids, such as foundation powders, lipsticks, and eye care;
15) Textile products, such as moisturizing or cleansing wipes; and
16) Pigments or dyes that impart color to the hair, skin, or textile substrate.

In accordance with the present invention, the household care ingredient must provide some benefit to the user. Examples of substances that may suitably be included, but not limited to, according to the present invention are as follows:
1) Perfumes, which give rise to an olfactory response in the form of a fragrance and deodorant perfumes which in addition to providing a fragrance response can also reduce odor;
2) Insect repellent agent whose function is to keep insects from a particular area or attacking skin;
3) Bubble generating agent, such as surfactant that generates foam or lather;
4) Pet deodorizer or insecticides such as pyrethrins that reduces pet odor;
5) Pet shampoo agents and actives, whose function is to remove dirt, foreign material and germs from the skin and hair surfaces;
6) Industrial grade bar, shower gel, and liquid soap actives that remove germs, dirt, grease and oil from skin, sanitizes skin, and conditions the skin;
7) All purpose cleaning agents, that remove dirt, oil, and grease, germs from the surface in areas such as kitchens, bathroom, and public facilities;
8) Disinfecting ingredients that kill or prevent growth of germs in a house or public facility;
9) Rug and Upholstery cleaning actives which lift and remove dirt and foreign particles from the surfaces and also deliver softening and perfumes;
10) A laundry softener active, which reduces static and makes fabric feel softer;
11) Laundry detergent ingredients which remove dirt, oil, grease, stains and kills germs;
12) Laundry or detergent or fabric softener ingredients that reduce color loss during the wash, rinse, and drying cycle of fabric care;
13) Dishwashing detergents which remove stains, food, germs;
14) Toilet bowl cleaning agents, which remove stains, kills germs, and deodorizes;
15) Laundry prespotter actives which helps in removing stains from clothes;
16) Fabric sizing agent which enhances appearance of the fabric;
17) Vehicle cleaning actives which removes dirt, grease, etc. from vehicles and equipment;
18) Lubricating agent which reduces friction between parts; and
19) Textile products, such as dusting or disinfecting wipes.

The above list of personal care and household care active ingredients are only examples and are not a complete list of active ingredients that can be used. Other ingredients that are used in these types of products are well known in the industry. In addition to the above ingredients conventionally used, the composition according to the present invention can optionally also include, but is not limited to, ingredients such as a colorant, preservative, antioxidant, nutritional supplements, alpha or beta hydroxy acid, activity enhancer, emulsifiers, functional polymers, viscosifying agents (such as salts, i.e., NaCl, NH₄Cl & KCl, water-soluble polymers, i.e., hydroxyethylcellulose, hydroxypropylmethylcellulose, and fatty alcohols, i.e., cetyl alcohol), alcohols having 1-6 carbons, fats or fatty compounds, antimicrobial compound, zinc pyrithione, silicone material, hydrocarbon polymer, emollients, oils, surfactants, medicaments, flavors, fragrances, suspending agents, and mixtures thereof.

In accordance with the present invention, examples of functional polymers that can be used in blends with the metal organic chelate treated cationic guar or derivatives thereof used in this invention include water-soluble polymers such as acrylic acid homopolymers such as Carbopol® product and anionic and amphoteric acrylic acid copolymers, vinylpyrrolidone homopolymers and cationic vinylpyrrolidone copolymers; nonionic, cationic, anionic, and amphoteric cellulosic polymers such as hydroxyethylcellulose, hydroxypropylcellulose, carboxymethylcellulose, hydroxypropylmethylcellulose, cationic hydroxyethylcellulose, cationic carboxymethylhydroxyethylcellulose, and cationic hydroxypropylcellulose; acrylamide homopolymers and cationic, amphoteric, and hydrophobic acrylamide copolymers, polyethylene glycol polymers and copolymers, hydrophobic polyethers, hydrophobic polyetheracetals, hydrophobically-modfied polyetherurethanes and other polymers referred to as associative polymers, hydrophobic cellulosic polymers, polyethyleneoxide-propylene oxide copolymers, and nonionic, anionic, hydrophobic, amphoteric, and cationic polysaccharides such as xanthan, chitosan, alginates and gum arabic.

In accordance with the invention, the silicone materials which can be used are polyorganosiloxanes that can be in the form of polymers, oligomers, oils, waxes, resins, or gums or polyorganosiloxane polyether copolyols, amodimethicones, cationic polydimethylsiloxane materials and any other silicone material that is used in personal care or household care compositions.

In one embodiment, the hair care or skin care composition is an aqueous system comprising water and the polymer of the invention. In one embodiment, the hair care or skin care composition contains one or more surfactant compounds, including amphoteric surfactants, cationic surfactants, anionic surfactants, nonionic surfactants, zwitterionic surfactants, and combinations thereof.

It has been found that titanium organic chelate treated cationic guars can deposit with high efficacy on hair/skin and can impart great conditioning benefits to the discussed keratin substrates.

Such polymers impart other benefits in hair styling, body lotions and sunscreens due to hydrophobic film formation on keratin substrates that would act as barrier between the these surfaces and the surrounding atmosphere.

The polymers used in this invention can be useful as conditioning agents in 2-in-1 shampoos, body lotions, sunscreens, antifrizz and hair styling. The polymers of this invention can also be used to improve hair volume, manageability, hair repair, or color retention, skin moisturization and moisture retention, fragrance retention, sunscreen longevity on hair, skin, and fabrics, flavor enhancement and antimicrobial performance in oral care applications, and improve fabric abrasion resistance and colorfastness in household care applications.

Wet and dry hair combability measurements are typical test methods used to measure conditioning performance in shampoo and conditioner applications. In skincare applications, skin lubricity or reduced friction or softer feel of the skin, reduced water vapor transmission and improved skin elasticity are test methods used to measure skin conditioning. In surfactant-based household cleansing product formulations where conditioning performance is desired, such as dish detergents, fabric softeners, and antistatic products, conditioning refers to imparting a softer feel to fabric and eliminating static effects, eliminating fabric fiber breakage or deformation known as pilling. Imparting color retention properties to fabrics is also important and can be measured.

The following examples demonstrate the crosslinking of cationic guar with organic titanium chelates (Tyzor^{®}LA organic titanates, supplied by E.I. du Pont de Nemours and Company) and their use in personal care compositions. In addition, it has been found that titanium organic chelate treated cationic guars function well as conditioning agents and thickening agents in personal care compositions.

The examples are merely set forth for illustrative purposes all parts and percentages being by weight, unless otherwise indicated. It is to be understood that other modifications of the present invention can be made by skilled artisans in the related industry without departing from the spirit and scope of the invention.

### EXAMPLES 1-6

### Cationic Guar Preparation

Cationic guar was prepared by known procedures, without the use of a crosslinking agent.

Guar splits (750g), and water (450g) were mixed in a stirred reactor under nitrogen. The reactor was pressurized with nitrogen and vented to remove oxygen. The reaction was conducted at a temperature between 30-50°C, after addition of 3-chloro-2hydroxypropyltrimethylammonium chloride (288g), followed by 250grams 25% sodium hydroxide. The reaction was cooled to room temperature. The reaction product is shown in Example 1, and it used for crosslinking experiments in Examples 3-7 in Table 1.

A similar reaction was conducted with addition of sodium borate tetrahydrate to crosslink the cationic guar. This reaction product is shown in Example 2.

### Titanium Crosslinking

The unpurified cationic guar reaction product was placed in a container and the specified quantity of Tyzor^{®} LA organic titanates, diluted with water in Examples 3, 4, 5, 6 is added to the splits while mixing with a hand held mixer over two minutes. The reaction was conducted at ambient temperature. For Examples 4 and 6, a diluted solution of glacial acetic acid was added to the splits using the same mixing procedure, prior to addition of the Tyzor^{®} LA organic titanates, solution.

For example 7, the cationic guar reaction product was placed in an ABBE blender., with the rotational speed set at 2 (85rpm). The system was evacuated to 10 in HG and pressurized with nitrogen to 10 psi, followed by a 2 minute hold. This procedure was repeated 5 times. After venting, 31.9 grams Tyzor LA(50% active, E.I Du Pont de Nemours, Wilmington, DE) in 369 grams water was added to the cationic guar solids(pH11.5) over 30 minutes, evacuating and pressurizing with nitrogen after each 50 ml addition. The temperature was maintained at 18-22C. After the final Tyzor addition, the mixture was held at 22°C and mixed for 30 minutes, under 10 psi nitrogen pressure. The stirring was stopped and the product recovered.

### Wash Procedure

A measured weight of the crosslinked or non - crosslinked polymer was placed in an 8 ounce jar and distilled water was added as specified for wash 1 in Table 1. After 30 - 40 minutes, the top water layer was decanted from the splits and measured. A second wash was performed using the specified quantity of water in Table 1, and the slurry remained at room temperature for 24 hrs.

If a top layer of water was present, it was decanted and measured as Wash 3 recovered. Note that there was a significant amount of water decanted from the second wash of Examples 2 (borate crosslink control), Example 5 and 6 (titanate crosslinking). The form of the polymer in Examples 2, 5, and 6 was as discrete particulates after Wash 2, indicating sufficient crosslinking had occurred to prevent polymer swelling during the wash step. The polymer in Example 1(no crosslinker control) formed a continuous gel plug after Wash 2, as no crosslinker was present, and the polymer was dissolving in the wash water. In Examples 3 and 4, the polymer was present as swollen, but discrete gel particles after wash 2, indicating some crosslinking had occurred, but an insufficient amount to prevent polymer swelling during the wash step.

Resuspension of the polymer phases in Examples 1-6 in a third wash step demonstrates that crosslinking occurred in Examples 2-6, with recovery of a decanted water phase and isolation of particulate polymer phase. Example 1 (no crosslinker control) yielded a swollen gel which continued to swell with further addition of water.

For Example 7, the product (660 parts by weight) was mixed with 3300 pbw water for 30-40 minutes using a mechanical stirrer. The product then settled to the bottom of the beaker, and the liquid phase was decanted. The solid product was dried 72hrs in a hood, with air draft, to a solids content of 87%. The product was chilled with dry ice, then ground through a 0.5mm then a 0.2mm screen in a fluidized bed drier. This product was then used to prepare the conditioning shampoo of Example 8 and bodywash of Example 10, in Tables 2 and 3, respectively.

These examples demonstrate the preparation of a water-dispersible polygalactomannan on treatment with sufficient amounts of titanium crosslinkers.

| **Table 1. Crosslinking of Cationic Guar** | | | | | | | |
|---|---|---|---|---|---|---|---|
| EXAMPLE | 1 | 2 | 3 | 4 | 5 | 6 | 7 |
| | No crosslinker control | Borate Control | | | | | |
| Cat guar(DS0.13; XA1232-62) | | | 47.08 | 25 | 25 | 25 | 399.08 |
| Cationic guar(DS0.13; 56% water) | | | 107 | 56.8181818 | 56.81818182 | 56.81818182 | 907 |
| Tyzor LA(50%) active | | | 0.4708 | 0.25 | 3.75 | 3.75 | 15.9632 |
| wt boron/guar | 0 | 0.003 | | | | | |
| wt Titanium/wt guar | 0 | 0 | 0.001598639 | 0.00159864 | 0.023979592 | 0.023979592 | 0006394558 |
| grams 50% Tyzor solution | | | 0.9416 | 0.5 | 7.5 | 7.5 | 31.9264 |
| grams water to dilute Tyzor | | | 50 | 27 | | | 400 |
| grams glacial acetic acid | | | | 0.87 | | 0.87 | 31.9264 |
| grams water to dilute acid | | | | 23 | | 23 | 368.0736 |
| rxn temp/C | | 34C | 20C | 20C | 20C | 20C | 20C |
| pH | | | 11.9 | 10.1 | 11.9 | 9.5 | 11.5 |
| moles Tyzor/moles guar | | | | | | | |
| pH after rxn | | | 11.2 | 9.57 | | | |
| Wash Step | | | | | | | |
| grams polymer | 17.2 | 9.3 | 12.3 | 9 | 13.4 | 14.4 | |
| wash 1 | 100 ml | 100 ml | 100 ml | 100 ml | 100ml | 100ml | |
| recovered wash 1(30 minutes) | 50ml | 50ml | 50ml | 50ml | 50ml | 50ml | |
| wash 2(24hr) | 70 | 70 | 60 | 70 | 90ml | 90ml | |
| recovered wash 2(24hr) | 0 (swollen gel) | 70ml (compact particulates) | 0 (swollen particulates) | 1 (swollen particulates) | 70ml(compac t particulates) | 70ml(compact particulates) | |
| wash 3(1hr) | 70ml | 70ml | 70ml | 70ml | 70ml | 70ml | |
| recovered wash 3(1 hr) | 0 | 70ml | 50ml | 50ml | 70ml | 70ml | |
| pH wash 2 | 11.9 | 12 | 11.9 | 9.6 | | | |

### EXAMPLE 8 AND COMPARATIVE EXAMPLES 9-10

### Demonstration of Conditioning Performance of Products of the Invention

The use of the cationic polygalactomannan materials of the invention of Example 7 in a conditioning shampoo formulation is demonstrated in Example 8, Table 2, and contrasted with a comparative control shampoo containing a borate crosslinked cationic guar of (Example 9) and a shampoo containing no cationic guar (Example 10).

### SHAMPOO PREPARATION

The conditioning shampoo formulations in Table 2 were prepared by combining 77 parts by weight (pbw) of the surfactant premix composition shown in Table 3 with 19 pbw deionized water, and 0.3 pbw of the polymer of the invention using a Caframo overhead mechanical stirrer with a dispersion blade, stirring at 600 rpm, and allowing the composition to mix for 45 minutes at ambient temperature. At this time, 3 pbw of a silicone emulsion (Dow Corning 1784) was added to the formulation, and mixing was continued for an additional 15 minutes. The shampoo compositions were maintained at ambient temperature overnight, and the viscosity of each shampoo was measured using a Brookfield LVT viscometer with a small sample adapter, spindle 31, at the specified rotation speed.

### Shampoo Viscosity Measurement

Comparison of the shampoo viscosity for Example 8, which contains the glyoxal crosslinked cationic guar of the invention, with comparative Example 10, which contains no polymer, demonstrates the viscosifying performance of the products of the invention. The viscosity of the shampoo in Examples 8 is similar to the viscosity of the shampoo containing borate crosslinked cationic guar in Example 9.

Cationic polysaccharides and other polymers have been used widely in personal care, household care, industrial, and institutional products to perform a function in the final product, ranging from the use of the polymer as gellants, binders, thickeners, stabilizers, emulsifiers, spreading and deposition aids and carriers for enhancing the rheology, efficacy, deposition, aesthetic and delivery of chemically and physiologically active ingredients in personal care, household care, institutional and industrial compositions. Depending on the application, the substrate to which the product is applied can be skin, hair, or textile substrates.

Cationic polysaccharides are used in hair care products to provide conditioning to the hair. In skin care products, these same polymers can provide conditioning effects to the skin. When incorporated into detergent and fabric softening formulations, these same polymers can provide conditioning, softening, anti-abrasion and antistatic characteristics to fabrics.

Wet and dry combability measurements are typical test methods used to measure conditioning performance in shampoo and conditioner applications. The combing performance of each shampoo formulation was measured within 24 hours of shampoo preparation, on two medium brown virgin European hair tresses (National Hair Importers, New Jersey) that had been previously treated with a solution of sodium lauryl sulfate (SLS), rinsed, and dried overnight at 23°C and 50% relative humidity.

### Combing Performance Measurements

Combing performance was measured by applying the shampoo formulation to a tress wet with water, at a ratio of 0.5pbw shampoo/l pbw hair tress. The tress was kneaded for 60 seconds, then rinsed with 40°C water for 30 seconds. This process was repeated, then the tress was rinsed with deionized water and excess water squeezed from the tress. The tress was placed on the double comb apparatus and wet combing force measured 8 times on an Instron 5542 at a cross head speed of 12.5cm/min using the double comb method, with Ace hard rubber fine pocket combs, at 23°C and 50% relative humidity. Hair tresses were then allowed to dry overnight at 23°C and 50% relative humidity, and the dry comb performance was measured using the same double comb method. The normalized comb energies in Table 2 represent the total comb energy/weight of tress.

The conditioning performance of the products of the invention is demonstrated by the significantly reduced wet and dry combing energy results for Example 8 compared to the higher combing energies for the no polymer control shampoo in Example 10. The combing energies for Examples 8 compare well with the comb energy for the shampoo containing borate crosslinked cationic guar in Example 9.

**Table 2. Titanium Lactate Crosslinked Cationic Galactomannan Polymers Performance in Conditioning Shampoo**

| Example | 7 | | |
|---|---|---|---|
| Treatment | Titanium Lactate | | |
| Cationic DS | 0.14 | | |
| % Moisture | 7.8 | | |
| Aqueous Viscosity @ 1% as received | 2650 | | |
| Silicone Shampoo Examples with Comb Energy on Virgin Medium Brown European Hair | | | |

| Example | 8 | 9(comparative example)¹ | 10(Comparative Example-No Polymer) |
|---|---|---|---|
| Conditioning Shampoo viscosity (Brookfield LVT sp. #31, small sample adapter, 6rpm, pH 5.1) | 3285 | 6010(3rpm;pH5.8) | 1421(12rpm) |
| Normalized Wet Comb Energy (gf-mm/g) | 1273 | 963 | 2340 |
| Normalized Dry Comb Energy (gf-mm/g) | 329 | 242 | 670 |

| | | | |
|---|---|---|---|
| 1. N-Hance® 3196 cationic guar, borate crosslinked (Aqualon Division of Hercules Incorporated) | | | |

**Table 3. Shampoo Premix Composition**

| Ingredient | manufacturer | Parts by weight(pbw) |
|---|---|---|
| Deionized water | | 896 |
| Stepanol AM | Stepan Company, Northfield, IL | 1027 |
| Steol CA-330 | Stepan Company | 310 |
| Amphosol CA | Stepan Company | 186 |
| Glydant | Lonza Group LTD, Basel Switzerland | 16.25 |
| 25wt% Ammonium Chloride(aq) | | 65 |

### EXAMPLES 11 and COMPARATIVE EXAMPLE 12

### Demonstration of use in Skin Care Application, Bodywash

The thickening performance of the products of the invention in a bodywash formulation are demonstrated in Table 4. Bodywash formulations were prepared by addition of 0.3 pbw of the polymers of the invention in Example 7 to 76 pbw of the bodywash premix formulation in Table 5, and water (added to bring the volume to 100). Mixing was performed using an overhead mechanical stirrer with a dispersion blade, for 1 hr. The pH of the bodywash was 5.6.

The bodywash example 11 contains the polymer of the invention of Example 7. Addition of the polymer of the invention to the bodywash formulation leads to increased viscosity of the bodywash relative to the comparative control bodywash, containing no cationic guar, in Example 12.

**Table 4. Performance of Products of the Invention in Bodywash Formulation**

| Example | 11 | 12 |
|---|---|---|
| Polymer | Ex. 7 | None |
| Viscosity/cps¹ | 3080(6rpm) | 1864 |

| | | |
|---|---|---|
| 1. Brookfield LVT, spindle3, 12 rpm | | |

**Table 5. Bodywash Premix Formulation**

| Ingredient | Manufacturer | Parts by weight |
|---|---|---|
| Stepanol AM | Stepan Company, Northfield, IL | 697 |
| Steol CA 330 | Stepan Company | 2500 |
| Amphosol CA | Stepan Company | 500 |
| Deionized Water | | 279 |
| Glydant | Lonza Group LTD, Basel Switzerland | 24.5 |

In addition to the use of non-borate metal salts as crosslinking agents for guar polymers and their derivatives, other agents which form a water-swellable or water-dispersible complex with the polygalactomannan polymers, can also act to improve the water-dispersibility of the polygalactomannan. These agents include oligomers or polymers containing phosphate, sulfate, sulfonate, carboxylate, or carbonate groups, including sodium hexametaphosphate polystyrene sulfonate, and proteins such as casein or whey which can form a water-dispersible complex with cationic polygalactomannan polymers. These agents also include anionic, cationic, and amphoteric surface-active agents such as ammonium lauryl sulfate, sodium lauryl sulfate, cetyltrimethylammonium chloride or bromide, and cocamidopropyl betaine.

In addition, other crosslinkers, such as chloroformate, siloxane based crosslinking reagents, such as triethoxysilane, glyoxal and other dialdehyde materials can be used to crosslink the polygalactomannan, rendering it water-dispersible.

The water-dispersible crosslinked products described above can then be used in applications such as personal care or household care products, where they can be dispersed and dissolved in aqueous phases by appropriate adjustment of the solution pH or by addition of salts.

Although the invention has been described with referenced to preferred embodiment, it is to be understood that variations and modifications in form and detail thereof may be made without departing from the scope of the claimed invention. Such variations and modifications are to be considered within the purview and scope of the claims appended hereto.

## Claims

1. Use of a non-borate titanium or zirconium chelate treated cationic guar having a substituent degree of substitution (DS) lower limit of 0.001 and an upper limit of 3.0 for personal care or household care.

2. The use of claim 1, wherein the non-borate titanium or zirconium chelate treated cationic guar has a weight average molecular weight (Mw) with a lower limit of 50,000 and an upper limit of 5,000,000.

3. The use of claim 1, wherein the personal care is selected from the group consisting of cleansing, conditioning, and hair styling.

4. The use of claim 3, wherein the cationic guar is used in combination with one or more surfactant compounds selected from amphoteric surfactants, cationic surfactants, anionic surfactants, nonionic surfactants, zwitterionic surfactants, and combinations thereof.

5. The use of claim 4, where the cationic guar is used in combination with one or more additional ingredients selected from the group consisting of preservatives, thickeners, functional polymers, viscosity modifiers, electrolytes, pH adjusting agents, perfumes, dyes, UV screens, organosilicone materials, antidandruff agents, vitamins, vitamin derivatives.

6. The use of claim 3, wherein the personal care is hair care.

7. The use of claim 3, wherein the personal care is skin care.

8. The use of claim 1, which is for household care.

## Patentansprüche

1. Verwendung eines kationischen Guar-behandelten Nicht-Borat-Titan- oder Zirkoniumchelats, welches einen Substitutionsgrad der Substitution (DS) mit einem unteren Limit von 0,001 und einem oberen Limit von 3,0 aufweist, zur Körperpflege oder Haushaltspflege.

2. Verwendung gemäss Anspruch 1, wobei das kationische Guar-behandelte Nicht-Borat-Titan- oder Zirkoniumchelat ein gewichtsgemitteltes Molekulargewicht (Mw) mit einem unteren Limit von 50.000 und einem oberen Limit von 5.000.000 aufweist.

3. Verwendung gemäss Anspruch 1, wobei die Körperpflege aus der Gruppe bestehend aus Reinigung, Konditionierung und Haarstyling ausgewählt ist.

4. Verwendung gemäss Anspruch 3, wobei das kationische Guar in Kombination mit ein oder mehreren Tensidverbindungen, ausgewählt aus amphoteren Tensiden, kationischen Tensiden, anionischen Tensiden, nicht-ionischen Tensiden, zwitterionischen Tensiden und Kombinationen davon, verwendet wird.

5. Verwendung gemäss Anspruch 4, wobei das kationische Guar in Kombination mit einem oder mehreren zusätzlichen Bestandteil(en), ausgewählt aus der Gruppe bestehend aus Konservierungsmitteln, Verdickungsmitteln, funktionellen Polymeren, Viskositätsmodifikatoren, Elektrolyten, pH-Einstellmitteln, Parfümes, Farbstoffen, UV-Filtern, Organosiliconmaterialien, Antischuppenmitteln, Vitaminen und Vitaminderivaten, verwendet wird.

6. Verwendung gemäss Anspruch 3, wobei die Körperpflege eine Haarpflege ist.

7. Verwendung gemäss Anspruch 3, wobei die Körperpflege eine Hautpflege ist.

8. Verwendung gemäss Anspruch 1, die zur Haushaltspflege ist.

## Revendications

1. Utilisation d'un guar cationique traité par un chélate de titane ou zirconium non-borate ayant un degré de substitution de limite inférieure de substitution (DS) de 0,001 et une limite supérieure de 3,0 pour les soins personnels ou l'entretien ménager.

2. Utilisation selon la revendication 1, dans laquelle le guar cationique traité par un chélate de titane ou zirconium non-borate a un poids moléculaire moyen en poids (Mw) avec une limite inférieure de 50 000 et une limite supérieure de 5 000 000.

3. Utilisation selon la revendication 1, dans laquelle le soin personnel est choisi dans le groupe constitué par un nettoyage, un conditionnement, et un coiffage des cheveux.

4. Utilisation selon la revendication 3, dans laquelle le guar cationique est utilisé en combinaison avec un ou plusieurs composés tensioactifs choisis parmi les tensioactifs amphotères, les tensioactifs cationiques, les tensioactifs anioniques, les tensioactifs non ioniques, les tensioactifs zwittérioniques, et les combinaisons de ceux-ci.

5. Utilisation selon la revendication 4, dans laquelle le guar cationique est utilisé en combinaison avec un ou plusieurs ingrédients supplémentaires choisis dans le groupe constitué par les conservateurs, les épaississants, les polymères fonctionnels, les modificateurs de viscosité, les électrolytes, les agents d'ajustement du pH, les parfums, les colorants, les filtres UV, les matériaux organosilicone, les agents antipelliculaires, les vitamines, les dérivés de vitamine.

6. Utilisation selon la revendication 3, dans laquelle le soin personnel est un soin des cheveux.

7. Utilisation selon la revendication 3, dans laquelle le soin personnel est un soin de la peau.

8. Utilisation selon la revendication 1, qui est pour un entretien ménager.
